# EUROPEAN PATENT SPECIFICATION

(11) **EP 0 794 280 B1**
(45) Date of publication and mention of the grant of the patent: **17.10.2007**
(21) Application number: 97660009.8
(22) Date of filing: 05.02.1997
(51) Int. Cl.: D06M 15/647, D06M 15/643, D06M 23/16, D04H 1/42

(54) **Finishing treatment method for nonwoven, a nonwoven fabric treated according to the method and a product containing the nonwoven fabric**
Verfahren zur Behandlung von Vliesstoffen, behandelte Vliesstoffe und Produkte mit diesen Vliesstoffen
Procédé de traitement de non-tissés, non-tissé traité, et produit le contenant

(30) Priority: 09.02.1996 FI 960602
(43) Date of publication of application: 10.09.1997
(73) Proprietor: Suominen Nonwovens Ltd., 29250 Nakkila (FI)
(72) Inventor: Hautojärvi, Joni, 29250 Nakkila (FI); Bergholm, Heikki, 00340 Helsinki (FI)
(74) Representative: Grew, Eva Regina

(56) References cited:
- EP-A- 0 546 580
- EP-A- 0 748 894
- US-A- 3 769 060
- US-A- 4 585 449
- PATENT ABSTRACTS OF JAPAN vol. 016, no. 342 (C-0966), 24 July 1992 -& JP 04 100981 A (KOREHOO:KK;OTHERS: 01), 2 April 1992,
- FIEDLER HERBERT P.: "LEXIKON DER HILFSSTOFFE FÜR PHARMAZIE, KOSMETIK UND ANGRENZENDE GEBIETE; BAND 2" 1996, EDITIO-CANTOR-VERLAG FÜR MEDIZIN UND NATURWISSENSCHAFTEN GMBH , AULENDORF Triton (TM) * page 1609 - page 1610 *

## Description

### Scope of the invention

The present invention relates to a finishing treatment method for nonwoven fabrics, in which method for specific areas of the nonwoven fabric is defined hydrophilic or hydrophobic properties, and the properties for at least one specific area are achieved by means of printing techniques, particularly by the flexographic printing techniques. The purpose of the method is a finishing treatment for nonwoven fibrous fabrics rendering the dry-feel comfort of the fabrics. The dry-feel comfort is achieved by means of a permanent hydrophilicity, which term is used in connection with this application to refer to a degree of hydrophilicity of the fabrics with sufficient persistence to endure at least three wetting cycles with a liquid.

### Background of the invention

Absorbing disposable sanitary products such as diapers are generally comprised of a liquid-permeable coverstock made from nonwoven fibrous material, a layer containing a superabsorbent and/or cellulosic fiber and a liquid-impermeable backing layer. The coverstock used as the surface layer of diapers typically is a nonwoven fabric made from polyolefin fibers such as polypropylene or polyethylene fibers. The most important liquid-handling properties of the coverstock are a fast pass-through rate of liquid combined with a good dry-feel comfort. Such a good dry-feel comfort presumes that while the liquid flows quickly through the coverstock, no reverse flow of the liquid occurs back to the surface of the coverstock fabric.

In order to make the inherently hydrophobic polyolefin fabric material used as the pass-through coverstock permeable to a water-based liquid, the material must be hydrophilized. Staple fiber made by carding and thermobonding nonwoven fibrous fabrics are hydrophilized in conjunction with the spinning process. Correspondingly, spun-bonded and melt-blown nonwoven fabrics are hydrophilized by functionally coating the bonded fabric with a wetting agent known as surfactant.

Typically, the one-way function of a coverstock selected to possess both good pass-through and good dry-feel comfort properties is based thereon that the hydrophilic functional coating materials, which are applied to the fiber or nonwoven fabric, are highly water-soluble surfactants. When passing through the nonwoven fabric, the absorbed liquid dissolves a major portion of the surfactant(s) applied to the nonwoven fabric, whereby the fabric changes hydrophobic at the pass-through point thus preventing the liquid from flowing back. Conventionally, the coverstock materials are made hydrophilic over their entire area even if the liquid load is imposed on selected areas of the coverstock surface only. The liquid passed through the coverstock migrates laterally in the absorbing layer thus also wetting those parts of the absorbent that are not located in the immediate vicinity of the liquid-loaded point. As the surfactant is essentially dissolved from the liquid-loaded point of the coverstock, the flow-back of the liquid from the absorbent will readily take place via the area of the coverstock which has remained hydrophilic. Moreover, dissolution of the surfactant lowers the surface tension of the liquid passed through the coverstock fabric thus aiding the flow-back of the liquid.

Such a one-time pass-through capability of the coverstock material, however, is not always satisfactory, but particularly in diapers, the coverstock fabric shall desirably be capable of passing the liquid successively a greater number of times. Such so-called permanently hydrophilic nonwoven fabric that can retain their absorbency for at least three successive pass-through cycles are hampered by their inferior dry-feel comfort. Since the nonwoven fabric stays hydrophilic for several pass-through cycles, it allows the liquid passed through the fabric to flow back to the surface of the coverstock.

The dry-feel comfort of absorbing disposable sanitary products can be improved, e.g., by adding between the absorbent and the coverstock material an intermediate layer of higher hydrophobicity than that of the coverstock (cf. US Pat. No. 4,798,603). Such an intermediate layer exhibiting a higher hydrophobicity than the coverstock isolates the absorbent from the coverstock thereby preventing the flow-back of the liquid. US Pat. No. 5,257,982 discloses a method in which the coverstock is thermobonded from a plurality of nonwoven fiber layers of different fiber diameters. Such a nonwoven fabric is made to have the smallest pore size on the coverstock inner surface facing the absorbent and the largest pore size on its outer surface. Thus, the large pores of the coverstock outer surface allow fast pass-through of a liquid, while the smaller pores of the inner side prevent flow-back of the liquid.

Correspondingly, US Pat. No. 5,273,596 describes a method for producing a thermobonded dual-layer nonwoven fabric having its one side comprised of both hydrophobic and hydrophilic fibers (such as polypropene and viscose rayon fibers, for instance), while the other side is principally comprised of hydrophobic fibers. Then, the hydrophobic side of the nonwoven fabric prevents liquid from flowing back from the absorbent to the hydrophilic outer side of the fabric.

A technically simpler and cheaper method for improving the dry-feel comfort properties of nonwoven fabric is to modify the liquid-handling capability of the fabric without altering the physical structure of the fabric. The dry-feel comfort of the coverstock can be improved, e.g., by reducing the proportion of the hydrophilic surface area of the nonwoven fabric by finishing the hydrophobic fabric so that the functional coating material is applied to selected areas of the fabric only (cf. EP Pat. No. 0611607 Al, US Pat. No. 4,585,449). Disclosed in US Pat. No. 4,585,449 is a coverstock material for a disposable sanitary product having, e.g., circular or rectangular surfactant-coated areas applied to its center. With regard to the state of the art, reference is made to patent publication EP 0611607 in particular, wherein a method is disclosed for functional coating of nonwoven fabric with a surfactant, or hydrophobicity-inducing agent, transferred to the fabric by means of a patterned roll. The surfactant can be applied, e.g., to the center of the nonwoven fabric, whereby the edge areas of the fabric remain hydrophobic.

The functional coating of nonwoven fabric may also be carried out using a printing press. Patent publication EP 0546580 A1 describes a functional coating for applying a homogeneous coating of a wetting agent, or surfactant, to nonwoven material by means of a gravure roll printer. By virtue of such printing technology, concentrated wetting agent solutions can be used, whereby the nonwoven material absorbs a smaller amount of the wetting agent solution than in other functional coating methods. Accordingly, the drying time of the coated fabric is shortened and the loss of fabric strength due to the drying is reduced.

It is an object of the present method to provide a functional coating method based on flexographic printing technology suitable for treating a fibrous nonwoven fabric to contain permanently hydrophilic areas of good dry-feel comfort.

### Summary of the invention

In the method according to the present invention, permanently hydrophilic functional coating material is applied to selected areas of the surface of a hydrophobic nonwoven coverstock fabric so that no significant amounts of said coating material will become migrated to the uncoated side of said nonwoven coverstock fabric. In the context of the invention, the term permanently hydrophilic functional coating material refers to a coating material with hydrophilic surface-chemical properties that make the material dispersible in water. Hence, such a coating material will not be dissolved in significant amounts to water, e.g., from a polyolefin surface, whereby the coverstock surface stays hydrophilic also in contact with water.

The present functional coating method may be applied to thermobonded, spun-bonded, melt-blown, water-jet bonded and other types of nonwoven fabrics that are intended for use as the coverstock material of absorbing disposable sanitary products.

The characteristics of the method according to the invention are disclosed in the attached claims.

The method according to the invention is based on the fact that the ink in the flexographic printing, well-known in the conventional colour printing, is replaced with a permanently hydrophilic functional coating material. Using a flexographic printing press, the coating material is applied to the surface of the nonwoven fabric so that, after printing, the nonwoven material surface will include areas containing said permanently hydrophilic coating material. The untreated fibrous nonwoven fabric used as the raw material is hydrophobic.

A particular benefit of the method according to the invention is the improvement of dry-feel comfort in the functionally coated, permanently hydrophilic nonwoven fabric. Furthermore, the surfactant can be applied on accurately defined areas to the surface of the nonwoven fabric, whereby only the area of the nonwoven fabric subjected to the major liquid-loading can be permanently hydrophilized. Thence, the method is also economical in use, because permanently hydrophilic functional coating materials are typically much more expensive than conventional surfactants. Accordingly, the application of the permanently hydrophilic coating material on those areas of the nonwoven fabric that are important for the end-use reduces the consumption of the coating material.

In the following a preferred embodiment of the invention is described in greater detail with reference to annexed drawings in which.
Figure 1 shows a roll of nonwoven fabric functionally coated according to one embodiment of the invention;
Figure 2 shows the coverstock of a diaper made from the roll of nonwoven fabric of Fig. 1;
Figure 3 is a diagrammatic view of the flexographic printing technique employed in the invention;
Figure 4 shows nonwoven fabric functionally coated by virtue of a preferred embodiment of the invention in a sequence of several flexographic printing units;
Figure 5 is a graph showing the pass-through times of nonwoven fabrics functionally coated according to example 1 as a function of pass-through cycles; and
Figure 6 is a graph showing the rewet times of nonwoven fabrics functionally coated according to Example 2 as a function of the liquid-loading factor.

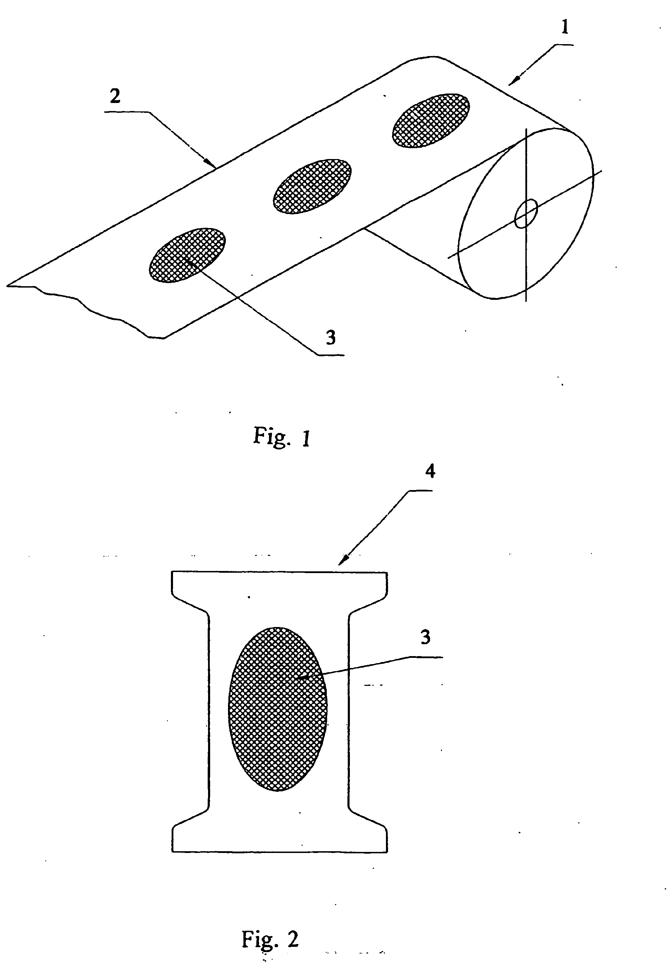

With reference to Fig. 1, the nonwoven material roll 1 shown therein, functionally coated according to the invention, is comprised of a nonwoven fabric material 2 containing polyolefin fibers such as a fibrous fabric well-known in the art being manufactured as thermobonded, spun-bonded, melt-blown or water-jet stitched nonwoven fabric. In addition to polyolefin fibers, a thermobonded or water-jet stitched nonwoven fabric may also contain other fibers such as polyester fibers. The nonwoven fabric basis material may be inherently hydrophobic, and its basis weight may vary in the range of 10-80 g/m². A hydrophobic nonwoven fabric is comprised of fibers whose outer surface forms a contact angle greater than 90° with water (e.g., polyolefin fibers).

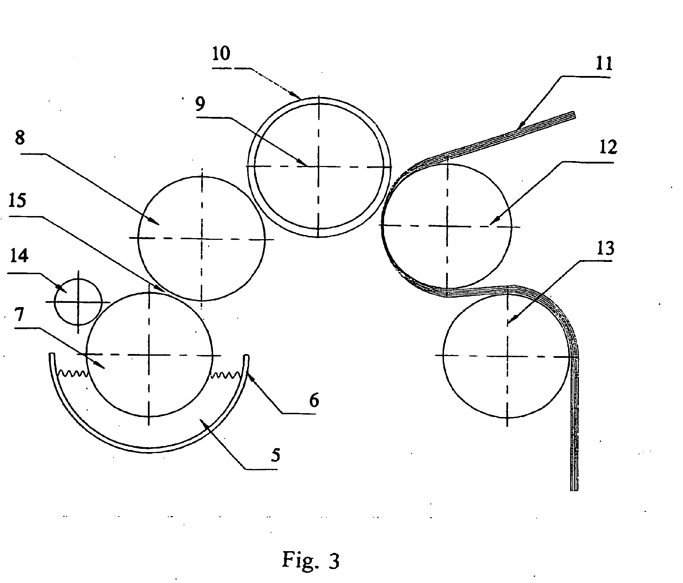

With further reference to Fig. 1, the nonwoven fabric shown therein, functionally coated according to a preferred embodiment of the invention, includes permanent hydrophilic surfactant-coated areas 3 which are processed using the flexographic printing technique illustrated in Fig. 3. The surfactant-coated areas 3 are advantageously applied to the printed surface of the nonwoven fabric so that the hydrophobicity properties on the uncoated side of the nonwoven fabric remain identical to those of the virgin nonwoven web 2. To render optimal dry-feel comfort properties to the coated nonwoven fabric, the basis material 2 is hydrophobic, whereby the uncoated side of the fabric remains entirely hydrophobic, and similarly the uncoated areas outside the coated areas 3 on the surfactant-coated side remain hydrophobic.

The nonwoven material roll 1 shown in Fig. 1 can be processed into diaper coverstock blanks 4 illustrated in Fig. 2. The diaper coverstock blank 4 is cut apart from the continuous web of the nonwoven fabric roll 1 so that the permanently hydrophilic area 3 is located at an advantageous place on the diaper.

The permanently hydrophilic surfactant-coated areas are printed to the nonwoven fabric using the flexographic printing technique illustrated in Fig. 3 known from the art of colour printing. The flexographic printing technique used in the invention will not be limited to the transfer roll printing unit described herein, but also other types of flexographic printing unit constructions such as a printing unit with a doctor-blade ink fountain are suitable for implementing the method according to the invention. The wetting agent solution or dispersion 5 is transferred from the ink pan 6 to the gravured anilox roll 8 by means of a transfer roll 7. The anilox roll wets the printing plate 10 which is wrapped about the press roll. To the printing plate 10 made of a photosensitive polymeric material is photo-engraved a raised pattern corresponding to the shape of the surfactant-coated areas to be applied to the nonwoven fibrous fabric material 11. The printing roll 9 with the printing plate 10 wrapped about it transfers the wetting agent to the surface of the nonwoven material 11 so that the mirrored shape of the raised pattern on the printing plate 10 is imprinted to the surface of the fabric. The path of the fabric in the printing unit is guided by a backing roll 12 and a pull roll 13. The amount of wetting agent applied to nonwoven material 11 is controlled by adjusting the nip pressure between the doctoring roll 14 and the transfer roll 7 as well as by the width of the gap 15 between the transfer roll 7 and the anilox roll 8. Also the viscosity of the coating agent solution 5 affects the amount of the printing solution transferred to the non-woven fabric.

The printing of the fabric with the permanently hydrophilic functional coating materials can be made using either water or organic solvents such as isopropanol, ethyl acetate or ethanol as the coating solvent. When the printing process is carried out using a coating solvent which does not wet the nonwoven material, a higher surface affinity of the printing pattern is attained, whereby the coating material adheres to the surface of the fabric without migrating further. Thence, it is most advantageous to use an aqueous dispersion of the coating material for printing to a hydrophobic nonwoven fabric. However, this requires that emulsifying agents are admixed with the water-insoluble coating material. Solvent-based printing formulations are advantageous in the case that a fast-drying printing solution is desirable. Moreover, solvents capable of wetting the nonwoven fabric at the printed area provide a more homogeneously coated surface than what is achievable by using aqueous dispersions of the coating material.

The dry-feel comfort properties of a permanently hydrophilic nonwoven fabric are dependent on the amount of coating material applied to the fabric. To achieve a sufficiently fast strike-through of the absorbed liquid in a hydrophobic nonwoven fabric, the permanently hydrophilic surfactant must be applied to the area being printed by at least about 0.2-0.5 wt.-% per unit weight of the non-woven basis fabric depending on the porosity structure and hydrophobicity of the fabric. Frequently, the coverstock material is expected to have a special property, namely, fast strike-through particularly during the first wetting cycle with a liquid. Then, the hydrophobic nonwoven fabric can be treated during its functional coating with a mixture of both a water-soluble surfactant and a permanently hydrophilic coating material. Owing to the surfactant component, the first-time strike-through of the fabric is fast and the permanently hydrophilic coating material component assures that the nonwoven fabric stays hydrophilic over a greater number of pass-through cycles.

Organomodified polysiloxane-based coating materials (such as silicone-ethyleneoxide copolymers, for instance) representing the most cost-effective permanently hydrophilic coating agents of the state of the art are generally detrimental to the thermobonding of polyolefin fibers and seamability of polyolefin-fiber nonwoven fabrics. For instance, thermobonded nonwoven fabrics made by carding from staple fiber treated with polysiloxane compounds typically have a strength inferior to that of fabrics coated with other types of finishing treatment materials. Particularly for fabrics made from staple fiber, the method according to the invention offers the significant benefit that the nonwoven fabric can be bonded prior to the coating treatment. Moreover, the seamability properties of nonwoven fabrics will not deteriorate in the coating process, because the seamed areas of the fabric can be left outside the permanently hydrophilic areas.

The invention is most advantageously implemented for carded, thermobonded nonwoven fabrics so that the fibers are in conjunction with the fiber spinning process treated with a hydrophobic antistatic agent and a water-dispersible lubricant, which lowers the kinetic fiber to fiber and fiber to metal friction. The treated, hydrophobic staple fiber is carded and thermobonded, whereafter the nonwoven fabric is coated on a flexographic printing press on selected areas with a permanently hydrophilic modified polydimethyl siloxane surfactant.

The printing plates of the flexographic printing process are made by exposing a printing plate of photosensitive polymer with UV light via an appropriately patterned negative. The exposure of the printing plate with UV light hardens the exposed areas of the plate, after which the unexposed areas can be dissolved away thus forming the desired raised pattern on the printing plate. Due to the simple processing of the printing plates, the printing patterns of the coating agent can be easily varied and modified in the functional coating method according to the invention. Furthermore, as a flexographic printing press typically includes at least four printing units, the same basis fabric web can be coated in a single run with both permanently hydrophilic and hydrophobic areas. Hence, the method makes it possible to apply with great accuracy a number of areas of different hydrophilicity/ hydrophobicity to the nonwoven fabric.

As the permanently hydrophilic or hydrophobic areas printed to the fabric web are colourless, further processing of the web roll requires the web to be printed with coloured alignment markings. Such alignment markings allow the web to be guided, e.g., in diaper-making machines so that the areas of the web coated in the printing press will be correctly aligned on the diaper. The permanently hydrophilic coating agent can be printed on one printing unit to the surface of the nonwoven fabric, while the coloured alignment marking is printed on another printing unit.

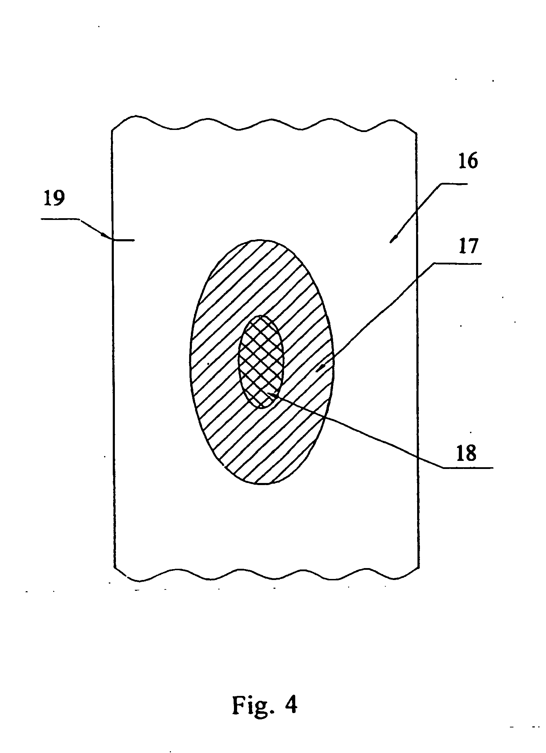

With reference to Fig. 4, therein is shown a nonwoven fabric coated according to a preferred embodiment of the invention using three printing units for the application of the coating. On the first printing unit, a hydrophilic area 17 is printed to a web of a hydrophobic nonwoven fabric 16. On a second printing unit on the hydrophilic area 17 is next applied a permanently hydrophilic area 18 on which the major portion of the liquid load in the end use of the nonwoven fabric is focused. A coloured alignment mark 19 printed on the third printing unit serves to guide the travel of the web in a diaper-making machine so that the areas 17 and 18 are aligned at desired points of the end product.

Nonwoven fabrics functionally coated according to the invention are suitable for use as the coverstock material of, e.g., absorbing disposable sanitary products. Particularly for the dry-feel comfort of diapers, it is most advantageous that the uncoated side of the nonwoven fabric is in intimate contact with the absorbent or intermediate layer of the diaper, while the permanently hydrophilic areas are located on the outer surface thereof. The liquid strike-through time of the coverstock material is substantially equal through both the coated surface and the uncoated surface. However, the dry-feel comfort properties of the coverstock material are clearly better if the liquid first passes through the coated surface of the nonwoven coverstock material. Then, the inner surface of hydrophilic coverstock, which is inherently hydrophobic, can prevent liquid flow-back from the absorbent to the upper surface of the coverstock. However, the permanently hydrophilic areas printed to the coverstock fabric retain their pass-through property for a greater number of times, whereby the pass-through capability of the coverstock material will not be substantially compromised, while the dry-feel comfort properties of the coverstock material is improved over conventional nonwoven fabrics treated homogeneously with a functional coating.

The examples described below elucidate the function of nonwoven fabrics treated according to the invention with regard to their strike-through time and dry-feel comfort properties. Testing of the liquid-handling characteristics of these nonwoven fabrics were carried out using the following methods:
**1. Repeated liquid strike-through time test:** The liquid strike-through time of the nonwoven fabrics (abbreviated as LSTT in Tables 1-4) was measured using the standard test method (Edana Recommended Test) ERT 150.2-93 (Liquid Strike-Through Time) of EDANA (European Disposables and Nonwovens Association). After the first value of strike-through time was measured, the blotting paper sheet was replaced and the strike-through time measurement was repeated at the same point of the nonwoven fabric. The measurement was repeated five times in sequence, whereby the increase of strike-through time as a function of the measurement cycle number is inversely proportional to persistence of the hydrophilicity of the nonwoven fabric. The hydrophilicity persistence of a nonwoven fabric is considered good if the strike-through time of the fifth measurement cycle is below 3.5 s.
**2. Liquid rewet:** This test was carried out according to the ERT 151.0-93 standard test (Coverstock Wetback) using loading factors in the range 3.5-3.8. Also the liquid strike-through time of the coverstock was simultaneously determined according to the above-mentioned ERT 150.2-93 test. The liquid rewet and strike-through time were measured for both the printing side and inner side of the nonwoven fabric.
**3. Surfactant content:** In this test the nonwoven fabrics were analyzed for the content of surfactant (g surfactant per g nonwoven) absorbed in the flexographic printing process using a procedure in which the surfactant was eluted from the nonwoven fabrics into carbon disulfide and then the polysiloxane content of the elution solution was measured by an IR spectrophotometer at the 1259 cm⁻¹ absorption wavelength of the Si-O bond.
**4. Hydrostatic head:** The hydrophobicity of untreated non-woven fabric was determined by capping the lower end of a 7.5 cm dia. column with the fabric. The column was gradually filled with 0.9 wt.-% NaCl aqueous solution so that the hydrostatic head of the liquid column imposed on the fabric increased at a rate of 3 cmH₂O/min. The hydrostatic pressure at which the nonwoven fabric first time starts to leak the solution is marked as the hydrostatic head value of the fabric.

In the examples, the liquid strike-through time and rewet tests were made at the functionally coated center area of the nonwoven fabrics. The test results given below are each an average value from three parallel measurements.

### Example 1

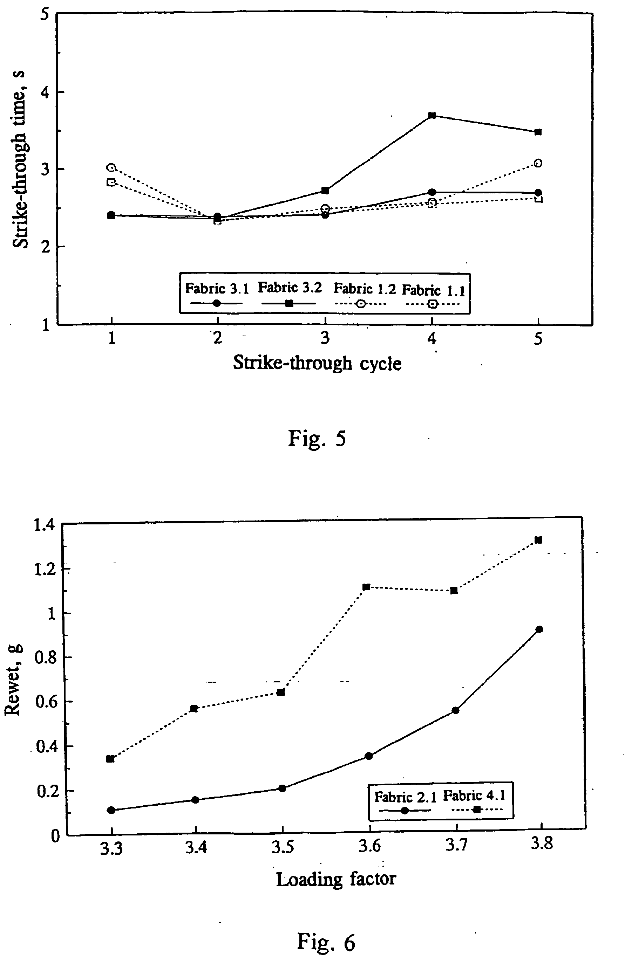

Using the transfer-roll-equipped printing unit of a PAVEMA flexographic printing press (manufactured by Maschinenbau Ludwig Meyer, Düsseldorf, Germany), to the center of a 38 cm wide, thermobonded nonwoven hydrophobic fabric made from carded polypropylene staple fiber with a basis weight of 33 g/m² was printed a continuous, 32.5 cm wide, permanently hydrophilic area. The liquid strike-through time of the unprinted nonwoven fabric was greater than 200 s and its hydrostatic head value was 99 mm as measured from the printing side of the fabric. Aqueous dispersions of organomodified polydimethyl siloxane in active agent concentrations of 20, 10 and 5 wt.-% were used as the printing solutions (on nonwoven fabric sample nos. 1.1, 1.2 and 1.3, respectively). The side (nappy side) of the nonwoven fabric, which had been facing the pin-patterned surface of the thermobonding roll, was used as the printing side of the fabric web. Five repeated cycles of liquid strike-through time measurements were made on the printing side of the nonwoven fabric. The rewet values and the liquid strike-through times determined by the same token were measured for both the printing side and the non-printed side. Table 1 lists the liquid strike-through time values of repeated measurements on the printing side of the nonwoven fabrics of Example 1 as well as the rewet and strike-through time values (at a loading factor of 3.7) from measurements performed on both sides of the nonwoven fabrics. Fig. 5 is a plot of the strike-through time values as a function of the measurement cycle number from repeated tests performed on the printing side of nonwoven fabric sample nos. 3.1 and 3.2. The same graph also shows the values of repeated strike-through time tests for the comparative samples of nonwoven fabric sample nos. 3.1 and 3.2. As is evident from Fig. 5, no substantial change appears in the values of repeated strike-through tests even if the coating solution is printed only to the outer surface of the coverstock fabric. The values given in Table 1 show further that no essential differences can be noticed in the strike-through times measured from the printed and unprinted sides of the fabric. By contrast, the rewet values for nonwoven fabrics with a good hydrophilicity persistence (that is, the fifth cycle value less than 3.5 s) are greater by an order of magnitude when measured from the un§printed side of the fabric.

### Example 2

Again using the transfer-roll-equipped printing unit of a PAVEMA flexographic printing press (manufactured by Maschinenbau Ludwig Meyer, Düsseldorf, Germany), to the center of a 67 cm wide, thermobonded nonwoven hydrophobic fabric made from carded polypropylene staple fiber with a basis weight of 30 g/m² was printed a continuous, 32.5 cm wide, permanently hydrophilic area. The liquid strike-through time of the unprinted nonwoven fabric was greater than 200 s and its hydrostatic head value was 91 mm as measured from the printing side of the fabric. Aqueous dispersions of organomodified polydimethyl siloxane in active agent concentrations of 40, 20 and 10 wt.-% having respectively 20, 10 and 5 wt.-% isopropanol added thereto as a solvent were used as the printing solutions (on non-woven fabric sample nos. 2.1, 2.2 and 2.3, respectively). The smooth side of the nonwoven fabric, which had been facing the smooth-surfaced thermobonding roll, was used as the printing side of the fabric web. Table 2 lists the liquid strike-through time values of repeated measurements on the printing side of the nonwoven fabrics of Example 2 as well as the rewet and strike-through time values (at a loading factor of 3.7) from measurements performed on both sides of the nonwoven fabrics. Fig. 6 is a plot of the rewet values as a function of the loading factor from tests performed on the printing side of nonwoven fabric sample no. 2.1 and the comparative sample of fabric sample no. 4.1.

As is evident from the values given in Table 2, the rewet values of the unprinted side of the fabric are greater by an order of magnitude in the same fashion as in Example 1 as compared to the values measured from the printed side of the fabric. Fig. 6 shows that the dry-feel comfort of a single-sidedly printed nonwoven fabric is clearly better than that of a comparative sample containing the same amount of the hydrophilic coating solution distributed homogeneously into the fabric.

### Example 3 (comparative samples)

A sample of the nonwoven fabric of Example 1 was treated with the aqueous emulsions of the active agent used in Example 1 so that the treated fabric contained 0.6, 0.3 and 0.15 wt.-% (fabric sample nos. 3.1, 3.2 and 3.3, respectively) of the active agent homogeneously distributed in the fabric. The same strike-through time and rewet tests as in Example 1 were performed on the nappy side of the nonwoven fabric. Table 3 shows the test results of the comparative example.

### Example 4 (comparative samples)

A sample of the nonwoven fabric of Example 2 was treated with the aqueous emulsions of the active agent used in Example 2 so that the treated fabric contained 3.5, 0.8 and 0.3 wt.-% (fabric sample nos. 4.1, 4.2 and 4.3, respectively) of the active agent homogeneously distributed in the fabric. The same strike-through time and rewet tests as in Example 2 were performed on the smooth side of the nonwoven fabric. Table 4 shows the test results of the comparative example.

**Table 1. Liquid strike-through time (LSTT) and rewet values of nonwoven fabrics tested in Example 1.**

| Nonwoven fabric | 1.1, 20wt.-% | 1.2, 10wt.-% | 1.3, 5wt.-% |
|---|---|---|---|
| Active agent conc. [wt.-%] | 0.64 | 0.32 | 0.15 |
| 1st cycle LSTT [s] | 2.8 | 3.0 | 3.7 |
| 2nd cycle LSTT [s] | 2.3 | 2.3 | 2.6 |
| 3rd cycle LSTT [s] | 2.4 | 2.5 | 2.7 |
| 4th cycle LSTT [s] | 2.6 | 2.6 | 3.6 |
| 5th cycle LSTT [s] | 2.6 | 3.1 | 5.2 |
| LSTT on printing side [s] | 2.7 | 3.1 | 4.1 |
| Rewet on printing side [g] | 0.29 | 0.16 | 0.18 |
| LSTT on inner side [s] | 2.7 | 2.7 | 3.9 |
| Rewet on inner side [g] | 1.55 | 0.67 | 0.13 |

**Table 2. Liquid strike-through time (LSTT) and rewet values of nonwoven fabrics tested in Example 2.**

| Nonwoven fabric | 2.1, 40wt.-% | 2.2, 20wt.-% | 2.3, 10wt.-% |
|---|---|---|---|
| Active agent conc. [wt.-%] | 3.5 | 0.83 | 0.31 |
| 1st cycle LSTT [s] | 2.6 | 5.9 | 39.9 |
| 2nd cycle LSTT [s] | 2.6 | 2.8 | 5.6 |
| 3rd cycle LSTT [s] | 2.6 | 2.7 | 4.2 |
| 4th cycle LSTT [s] | 2.6 | 2.9 | 4.3 |
| 5th cycle LSTT [s] | 2.9 | 3.7 | 5.3 |
| LSTT on printing side [s] | 2.4 | 7.6 | 59.0 |
| Rewet on printing side [g] | 0.54 | 0.18 | 0.15 |
| LSTT on inner side [s] | 2.5 | 3.0 | 6.2 |
| Rewet on inner side [g] | 1.46 | 1.44 | 0.11 |

**Table 3. Liquid strike-through time (LSTT) and rewet values of nonwoven fabrics tested in Example 3 (comparative example).**

| Nonwoven fabric | 3.1 | 3.2 | 3.3 |
|---|---|---|---|
| Active agent conc. [wt.-%] | 0.6 | 0.3 | 0.15 |
| 1st cycle LSTT [s] | 2.4 | 2.4 | 3.1 |
| 2nd cycle LSTT [s] | 2.4 | 2.4 | 2.9 |
| 3rd cycle LSTT [s] | 2.4 | 2.7 | 3.2 |
| 4th cycle LSTT [s] | 2.7 | 3.7 | 3.6 |
| 5th cycle LSTT [s] | 2.7 | 3.5 | 4.0 |
| LSTT [s] | 2.1 | 2.4 | 3.0 |
| Rewet [g] | 0.60 | 0.31 | 0.10 |

**Table 4. Liquid strike-through time (LSTT) and rewet values of nonwoven fabrics tested in Example 4 (comparative example).**

| Nonwoven fabric | 4.1 | 4.2 | 4.3 |
|---|---|---|---|
| Active agent conc. [wt.-%] | 3.5 | 0.8 | 0.3 |
| 1st cycle LSTT [s] | 4.8 | 2.7 | 3.7 |
| 2nd cycle LSTT [s] | 2.8 | 2.5 | 3.2 |
| 3rd cycle LSTT [s] | 2.4 | 3.1 | 4.1 |
| 4th cycle LSTT [s] | 2.6 | 3.5 | 5.8 |
| 5th cycle LSTT [s] | 2.6 | 4.2 | 6.0 |
| LSTT [s] | 7.2 | 2.7 | 3.5 |
| Rewet [g] | 1.08 | 0.13 | 0.11 |

## Claims

1. A finishing treatment method for hydrophobic nonwoven fabric for use in absorbing disposable sanitary products, in which method surfactant treated areas are accurately defined to the surface of the nonwoven fabric by means of printing techniques, **characterized in that**, of the said areas at least one is defined with surfactant with hydrophilic surface chemical properties, but being dispersible in water, and that the surfactant for the at least one area is applied only to the areas of the nonwoven fabric subjected to the major liquid-loading in the end use of the nonwoven fabric and **in that** in said printing is used such an amount of the surfactant that the printing effects the printing side of the non-woven fabric only.

2. A method as defined in claim 1, **characterized in, that** the nonwoven fabric used in the method is comprised of polyolefin fibers.

3. A method as defined in claims 1-2, **characterized in, that** the nonwoven fabric used in the method is produced by a thermobonding, spin-bonding, melt-blowing or water-jet stitching process and the basis weight of the fabric is in the range 10-80 g/m².

4. An absorbing disposable sanitary product comprising a liquid-impermeable backing layer, an absorbent layer and at least one layer of the nonwoven fabric treated according to the method defined in claims 1-3.

## Patentansprüche

1. Verfahren zur Endbehandlung von hydrophobem Vliesstoff zur Verwendung in absorbierenden Einweg-Sanitärprodukten, in welchem mit grenzflächenaktivern Mittel behandelte Bereiche an der Oberfläche des Vliesstoffes durch Drucktechniken genau definiert sind, **dadurch gekennzeichnet, dass** von den Bereichen mindestens einer durch ein grenzflächenaktives Mittel mit hydrophilen chemischen Oberflächeneigenschaften definiert ist, das aber in Wasser dispergierbar ist, und dass das grenzflächenaktive Mittel für den mindestens einen Bereich nur auf die Bereiche des Vliesstoffes aufgetragen wird, welche der Hauptflüssigkeitsaufnahme im Endgebrauch des Vliesstoffes unterliegen, und dass für das Drucken nur eine solche Menge des grenzflächenaktiven Mittels verwendet wird, dass das Drucken nur die Druckseite des Vliesstoffes betrifft.

2. Verfahren wie in Anspruch 1 definiert, **dadurch gekennzeichnet, dass** der im Verfahren verwendete Vliesstoff Polyolefinfasern umfasst.

3. Verfahren wie in den Ansprüchen 1 bis 2 definiert, **dadurch gekennzeichnet, dass** der im Verfahren verwendete Vliesstoff durch eine Wärmebindung, Spinbindung, Schmelzblasen oder durch ein Wasserstrahlen-Stichverfahren hergestellt wird und das Grundgewicht des Stoffes im Bereich von 10 bis 80 g/m² liegt.

4. Absorbierendes Einweg-Sanitärprodukt, umfassend eine flüssigkeitsundurchlässige Trägerschicht, eine Absorptionsschicht und mindestens eine Schicht des Vliesstoffes, behandelt nach dem Verfahren wie in den Ansprüchen 1 bis 3 definiert.

## Revendications

1. Procédé de traitement de finition de non-tissés hydrophobes utilisables dans des produits hygiéniques absorbants jetables, dans lequel des régions traitées par un agent tensioactif sont définies avec précision à la surface du non-tissé par des techniques d'impression, **caractérisé en ce que**, parmi les dites régions, au moins une est définie avec un agent tensioactif possédant des propriétés chimiques de surface hydrophiles mais pouvant être dispersé dans l'eau, et **en ce que** l'agent tensioactif pour la dite au moins une région est appliqué seulement aux régions du non-tissé qui sont exposées à la plus grande charge de liquide dans l'utilisation finale du non-tissé, et **en ce que**, dans la dite impression, on utilise une quantité de l'agent tensioactif telle que l'impression agit seulement sur le côté d'impression du non-tissé.

2. Procédé selon la revendication 1, **caractérisé en ce que** le non-tissé utilisé dans le procédé est constitué de fibres de polyoléfine.

3. Procédé selon les revendications 1 et 2, **caractérisé en ce que** le non-tissé utilisé dans le procédé est produit par un procédé de thermo-liaison, liaison en rotation rapide, soufflage à l'état fondu ou piqûre au jet d'eau, et le poids de base du non-tissé est dans la plage de 10 à 80 g/m².

4. Produit hygiénique absorbant jetable comprenant une couche de support imperméable aux liquides, une couche absorbante et au moins une couche du non-tissé traité conformément au procédé défini dans les revendications 1 à 3.
